(19) Europäisches Patentamt
European Patent Office
Office européen des brevets

(11) **EP 3 492 004 A1**

(12) **EUROPEAN PATENT APPLICATION**
published in accordance with Art. 153(4) EPC

(43) Date of publication:
**05.06.2019 Bulletin 2019/23**

(51) Int Cl.:
*A61B 5/02* *(2006.01)*    *G06F 19/00* *(2018.01)*

(21) Application number: **17833628.5**

(22) Date of filing: **28.03.2017**

(86) International application number:
**PCT/ES2017/070181**

(87) International publication number:
**WO 2018/020064 (01.02.2018 Gazette 2018/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.07.2016 ES 201631026**

(71) Applicant: **Universitat Politècnica De Catalunya 08034 Barcelona (ES)**

(72) Inventors:
• **PALLÀS ARENY, Ramon**
  **08034 Barcelona (ES)**
• **CASANELLA ALONSO, Ramon**
  **08034 Barcelona (ES)**
• **GÓMEZ CLAPERS, Joan**
  **08012 Barcelona (ES)**

(74) Representative: **Juncosa Miró, Jaime et al Torner, Juncosa i Associats, S.L. Gran Via de les Corts Catalanes, 669 bis, 1o, 2a 08013 Barcelona (ES)**

(54) **METHOD AND DEVICE FOR DETECTING MECHANICAL SYSTOLIC EVENTS FROM A BALISTOCARDIOGRAM**

(57)    A method and apparatus to detect systolic mechanical events from the ballistocardiogram (BCG) is proposed. First, a transfer function able to compensate for the mechanical response of the body of a subject is applied to a BCG of the same subject, in such a way that the overall transfer function is flat within the range of frequencies of interest. Thus, the resulting signal corresponds only to the mechanical events occurred at the heart and the aortic root, and the systolic mechanical events can be reliably detected from fiducial points on said resulting signal.

Figure 2

## Description

## FIELD OF THE INVENTION

The present invention relates in general to systems for measuring physiological parameters through physical methods and, more specifically, to a method and apparatus for detecting mechanical systolic events from the ballistocardiogram (BCG).

## BACKGROUND OF THE INVENTION

Systolic events are especially helpful in the non-invasively assessment of the health status of the heart. The intervals between systolic events, the so-called systolic time intervals (STI), provide information about electrical and mechanical parameters directly involved in the systolic activity and therefore their use as a diagnosis tool is widely established among the medical community. Some of the most interesting STI are the pre-ejection period (PEP), the delay between the electrical activation of the ventricles and the blood ejection into the aorta, and the left ventricular ejection time (LVET), the time interval between the opening and closure of the aortic valve. From a clinical perspective, a prolonged PEP is often attributed to decreased myocardial contractility or to decreased vascular elasticity whereas a shortened LVET reveals vascular deterioration and myocardial weakening. Consequently, the quotient PEP/LVET is commonly used, together with STI values, to assess the health status of the heart as described in the document by S. S. Ahmed, G. E. Levinson, C. J. Schwartz, and P. O. Ettinger, "Systolic Time Intervals as Measures of the Contractile State of the Left Ventricular Myocardium in Man," Circulation, DOI 10.1161/01.CIR.46.3.559.

Systolic events such as the closure of the aortic valve can also be used as a proximal site in pulse transit time (PTT) measurements to a more distal site, which reflect the elasticity of the arteries and are commonly used to assess the health status of the circulatory system. Particularly, the aortic PTT is associated to the presence of cardiovascular (CV) risk factors and atherosclerotic disease, and it has proved to be an excellent predictor of risk of future fatal and nonfatal CV events such as myocardial infarction, cerebral stroke, revascularization, heart stroke, or aortic syndromes, and total mortality as described in the document by C. Vlachopoulos, K. Aznaouridis, and C. Stefanadis, "Prediction of Cardiovascular Events and All-cause Mortality With Arterial Stiffness: a Systematic Review and Meta-analysis," Journal American College Cardiology, DOI 10.1016/j.jacc.2009.10.061. Further, PTT can be used to assess arterial pressure changes and for absolute arterial pressure measurements using different calibration methods as described in the document by D. Buxi, J. M. Redouté, and M. R. Yuce, "A Survey on Signals and Systems in Ambulatory Blood Pressure Monitoring Using Pulse Transit Time," Physiological Measurements, DOI 10.1088/0967-3334/36/3/R1.

Systolic events can be measured by non-invasive means on clinical settings using the electrocardiogram (ECG), which reflects the electrical activity of the heart and can be used to determine the electrical activation of the ventricles, and the Doppler echocardiogram, which can be used to identify mechanical events such as the aperture and closure of the aortic valve. Even though the ECG can be easily obtained from surface electrodes, the measurement of mechanical systolic events using a Doppler echocardiograph requires a skilled operator and the exposure of the thoracic area and its preparation with an aqueous gel, which entails a lengthy measurement procedure and discomfort for the subject.

The Doppler echocardiograph can be obviated by using a phonocardiograph and a pulse pressure sensor placed on the carotid artery. Using this setup, the closure of the aortic valve is directly indicated by the onset of the S2 sound on the phonocardiogram (PCG), the LVET can be measured on the carotid pulse signal, hence the aperture of the aortic valve can be calculated by subtracting LVET from the onset of S2 as described in the document by A. M. Weissler, W. S. Harris, and C. D. Schoenfeld, "Systolic Time Intervals in Heart Failure in Man," Circulation, DOI 10.1161/01.CIR.37.2.149. Even though this method avoids the exposure of the thoracic area, sensor placement is still cumbersome, especially that of the pressure pulse sensor on the carotid artery, and the procedure is in disuse.

An alternative method to detect mechanical systolic events is by using the impedance cardiogram (ICG), which is the recoding of changes in thoracic impedance measured between two pairs of electrodes usually placed at the base of the neck and around the chest at the height of the xiphosternal line as described in the document by L. Jensen, J. Yakimets, and K. K. Teo, "A Review of Impedance Cardiography," Hear. Lung J. Acute Crit. Care, DOI 10.1016/S0147-9563(05)80036-6. Even though this method to detect mechanical systolic events is relatively simple, it requires the exposure of the thoracic area and the placement of adhesive electrodes, which entails a lengthy measurement procedure and subject discomfort.

An alternative method to obtain information about mechanical activity derived from systolic activity and that requires less subject preparation is from fiducial points of the seismocardiogram (SCG), commonly obtained from an accelerometer placed on the thorax as described in the document by O. T. Inan, P. F. Migeotte, K.-S. Park, M. Etemadi, K. Tavakolian, et al., "Ballistocardiography and Seismocardiography: a Review of Recent Advances," IEEE Journal of Biomedical Health and Informatics, DOI 10.1109/JBHI.2014.2361732. Even though placing an accelerometer on the thorax is easier than fixing electrodes and can be performed on the clothing, the position of the sensor strongly affects the shape and amplitude of the measure signal, and its orientation can cause a crossing between measurement axes, which increases measurement uncertainty.

An alternative method to obtain information about mechanical activity resulting from the systolic activity and that does not require of the placement of sensors on the body of the subject and is less prone to crossings between measurement axes is from fiducial points of the ballistocardiogram (BCG), which reflects changes in the center of gravity of the human body as a result of blood ejection from the heart into the aorta during systole and the posterior propagation of the pressure pulse through the arterial tree. The BCG can be obtained from various setups, some of them implemented with sensors embedded in daily-use objects such as bodyweight scales, chairs, seats, or beds as described in the document by O. T. Inan, P. F. Migeotte, K.-S. Park, M. Etemadi, K. Tavakolian, et al.. Since no sensors are applied to the body but it is the body itself that naturally contacts with an element (platform, body weighing scale, chair, seat, bed, or an accessory used together with these objects such as a carpet, pillow, seat cover, etc.) where the BCG sensors are embedded, the BCG can be obtained fast and comfortably and, in some cases, for long time periods. However, even though the I and J waves of the BCG have been already used in some setups to indirectly detect changes on the aortic valve aperture moment to estimate the PEP (from the ECG Q wave), these waves are significantly posterior to the aperture of the aortic valve and their use for this purpose needs a calibration to correct for their delays. Further, no fiducial point has yet been identified in the BCG as a plausible indicator of the closure of the aortic valve.

The health status of the heart could be assessed faster, more comfortably, and for longer time periods thus enabling continuous monitoring, if the mechanical systolic events were detected from the BCG. The method would also be of great interest to calculate other health indicators that involve mechanical systolic events such as the PTT, to assess arterial stiffness, or arterial blood pressure.

## SUMMARY OF THE INVENTION

The present invention comprises a method and apparatus for detecting mechanical systolic events from the ballistocardiogram (BCG). The innovative solution proposed in the present invention is to apply a transfer function to the raw BCG of a subject to compensate for the mechanical response of the body of this subject in such a way that the signal belonging to the mechanical activity at the heart and aortic root can be reconstructed, and to detect fiducial points that allow us to identify the opening and closure of the aortic valve on said reconstructed signal. Since the BCG is commonly obtained from sensors embedded into a single element that naturally contacts the body of the subject, the use of the BCG obviates the placement of one or several sensors on his/her body.

This innovative solution relies on the assumption that the BCG waves result from cardiac ejection on every heartbeat and propagate through the body of the subject, and this implies that the recorded signal includes, in addition to those waves, the response of the body of the subject, which hinders the reliable observation of mechanical systolic events actually occurring in the heart and aortic root. Consequently, the method proposed in this invention consists, first, in applying to the BCG a transfer function to compensate for the mechanical response of the body of the subject in such a way that the global transfer function is flat and with zero phase within the range of frequencies of interest, usually between 0.5 Hz and 50 Hz, leaving on the resulting reconstructed signal only the contributions of the mechanical events produced at the heart and the aortic root.

A commonly accepted mechanical response of the body of subjects standing on BCG systems embedded on weighing scales is a second-order low-pass filter with a resonant frequency $f_r$ and damping factor $k_d$ that depend on the subject itself and have an approximate average value of 5 Hz and 0.2, respectively. A transfer function able to compensate the effect of said mechanical response could be, for instance, one with two zeros on the poles of the mechanical response, at

$$\left(2\pi f_r k_d,\ \pm j2\pi f_r\sqrt{1 - k_d^2}\right)$$ on the pole-zero plot, and two poles at the upper cutoff frequency $f_c$, at $(2\pi f_c, 0)$. Different methods can be applied to obtain the exact values of the parameters $f_r$ and $k_d$ that characterize the mechanical response of the body of a particular subject. For instance, the parameters could be obtained by comparing biometric data of the subject to statistical data obtained from a reference group of subjects, or be estimated from the signal recorded as a response to a maneuver performed by the subject itself. The result of applying said transfer function to a BCG recording is a signal corresponding to the mechanical activity occurred at the heart and the aortic root where two groups of waves B1 and B2 can be identified to be further used as fiducial points that belong to the aperture and closure of the aortic valve.

Even though the proposed method could be implemented by an expert able to reconstruct the signal corresponding to the mechanical activity occurred at the heart and the aortic root from the BCG of a subject and an estimation of the mechanical response of the body of the same subject, and to visually identify the fiducial points corresponding to systolic mechanical events, an optimal implementation of the proposed method would be by means of an apparatus containing a signal processing module able to apply to a BCG a transfer function that compensates for the mechanical response of the body of the subject and automatically locates said fiducial points on the resulting signal, and a communication system able to represent the result on a display or to communicate it to another device.

The main advantage of the invention here described is that it enables the detection of systolic mechanical events using only the BCG, thus avoiding the use of a Doppler echocardiograph or a phonocardiograph, which allows easier, faster and more comfortable measurements even during long time periods as compared to other commonly used systems.

**BRIEF DESCRIPTION OF THE DRAWINGS**

A clear conception of the advantages and features that constitute inventive arrangements, and of various construction and operational aspects of typical mechanisms provided by such arrangements, are readily apparent by referring to the following illustrative, exemplary, representative, and non-limiting figures, which form an integral part of this specification, in which like numerals generally designate the same elements in the several views, and in which:

Figure 1 - Shows a diagram of a bodyweight scale able to obtain the BCG, which constitutes the element with which the subject comes into contact in one of the embodiments of the present invention.

Figure 2 - Shows, from top to bottom, a recording of the ECG, the BCG obtained from a bodyweight scale, the signal corresponding to the mechanical activity occurred at the heart and the aortic rood, and the PCG, all of them simultaneously obtained from the same subject.

Figure 3 - Shows a recording of the BCG obtained from a bodyweight scale during a maneuver to estimate the mechanical response of the body of a subject, from which the resonance frequency and the damping factor can be measured.

Figure 4 - Shows an example of the mechanical response of the body of a subject, the transfer function applied to compensate for it, and the overall transfer function.

**DETAILED DESCRIPTION OF VARIOUS PREFERRED EMBODIMENTS**

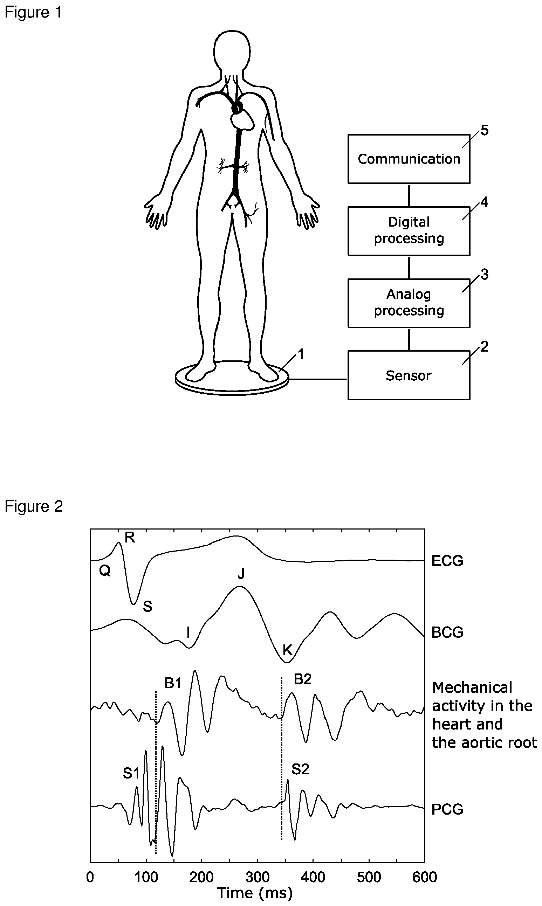

In a preferred embodiment of the invention, shown in Figure 1, a system embedded into a bodyweight scale (1) obtains a longitudinal BCG (on the head-feet axis) indicative of the mechanical activity related to cardiac ejection, from a sensor (2) constituted by the same strain gauges from which the body weight is measured on the scale, and an analog signal processing circuit (3).

From the BCG obtained at the output of the system described, a digital signal processing system (4) applies a transfer function that compensates for the mechanical response of the body of the subject in such a way that the overall transfer function is flat and with zero phase shift within the range of frequencies of interest, from 0.5 Hz to 50 Hz, and the resultant signal corresponds only to the mechanical activity occurred at the heart and the aortic root. In this preferred embodiment, the mechanical response of the body is modeled by a second-order low-pass filter with constant values of resonant frequency $f_r$ = 5 Hz and damping factor $k_d$ = 0.2 so that the applied transfer function has two zeros coincident with the poles of the low-pass filter and a double pole at the upper cutoff frequency $f_c$ = 50 Hz. After that, the digital signal processing system (4) detects fiducial points at the obtained signal that enable the identification of the aperture and closure of the aortic valve, which in this embodiment correspond to the onset of the groups of waves B1 and B2, respectively. Finally, a communication module (5) displays the measured values on an LCD screen.

Figure 2 shows simultaneous recordings of the ECG, the BCG, and the signal corresponding to the mechanical activity occurred at the heart and the aorta obtained from the same subject on this embodiment, wherein the groups of waves generated at the opening (B1) and closure (B2) of the aortic valve are clearly identifiable and their onsets are used as fiducial points to detect the aperture and closure of the aortic valve. A simultaneous PCG is also provided for reference. Figure 2 illustrates how the onset of group B1 occurs before the I wave of the BCG and during the S1 sound of the PCG, as expected for the aperture of the aortic valve, and how the onset of the group B2 occurs at the onset of the S2 wave of the PCG, indicative of the closure of the aortic valve.

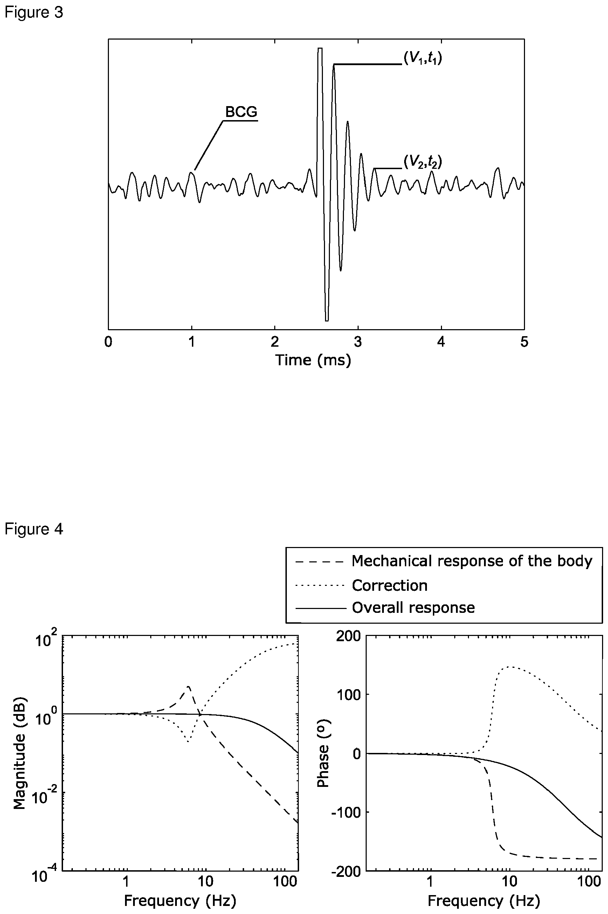

In order to have a more accurate estimation of the mechanical response of the body of a subject, on a second embodiment of this invention said estimation is from the system response to a maneuver performed by the subject, in which the subject softly hits the surface of the bodyweight scale with one or both heels. Figure 3 shows an example of the signal obtained during said maneuver, from which the resonant frequency $f_r$ is determined using the equation

$$f_r = \frac{N}{t_2 - t_1} \; ,$$

where $t_1$ and $t_2$ are the timings of two peaks and $N$ is the number of complete cycles between them, and the damping factor $k_d$ is determined using the equation

$$k_d = \frac{\log(V_1 - V_2)}{2\pi N} \; ,$$

where $V_1$ y $V_2$ are the peak value of said cycles.

Figure 4 shows the mechanical response of the body calculated from $f_r$ and $k_d$, the transfer function applied to compensate for it, and the total frequency response, which is flat and with zero phase shift within the range of frequencies of interest.

Once the invention has been sufficiently described, as well as two preferred embodiments, it should only be added that it is possible to make modifications in its constitution, materials used, and in the choice of the sensors used to obtain the BCG and in the methods to identify the fiducial points on the signal corresponding to the mechanical activity occurred at the heart and the aortic root, without deviating from the scope of the invention, defined in the following claims.

**Claims**

1. A method to detect systolic mechanical events from the ballistocardiogram (BCG) of a subject **characterized in that**:

   a) a transfer function that compensates for the mechanical response of the body of a subject is applied to the BCG of said subject in such a way that the overall transfer function is flat within the range of frequencies of interest
   b) systolic mechanical events are detected from fiducial points of the signal that results from applying said transfer function to the BCG.

2. A method according to claim 1 wherein the mechanical response of the body of the subject is modeled as a mechanical second-order low-pass filter.

3. A method according to claim 2 wherein the resonant frequency is modeled as a constant value between 4 Hz and 8 Hz, and the damping factor is modeled as a constant value between 0.5 and 0.005.

4. A method according to claims 1-2 wherein the parameters of the mechanical response of the body of the subject are calculated from biometrical data from said subject with respect to statistical data obtained from a group of subjects of reference.

5. A method according to claims 1-2 wherein the parameters of the mechanical response of the body of the subject are calculated from data obtained from a maneuver performed by the user.

6. A method according to claims 1-2, and 3, 4 or 5, wherein fiducial points in the signal obtained after the filter are used to detect the aperture of the aortic valve.

7. A method according to claim 6, wherein the aperture of the aortic valve is detected from the onset of the group of waves B1.

8. A method according to claims 1-2, and 3, 4 or 5, wherein fiducial points in the signal obtained after the filter are used to detect the closure of the aortic valve.

9. A method according to claim 6, wherein the closure of the aortic valve is detected from the onset of the group of waves B2.

10. An apparatus to automatically detect systolic mechanical events from the ballistocardiogram (BCG) of a subject, comprises:

    a) a signal processing system able to apply a transfer function that could compensate for the mechanical response of the body of the subject in such a way that the overall transfer function is flat in the range of frequencies of interest to the BCG of said subject;
    b) a signal processing system able to detect fiducial points on the signal obtained after applying said transfer function to the BCG of the subject and to identify the aperture and closure of the aortic valve from said fiducial points;
    c) a communication system able to communicate the obtained result to a user or to another device.

Figure 1

Figure 2

Figure 3

Figure 4

EP 3 492 004 A1

# INTERNATIONAL SEARCH REPORT

| | International application No. |
|---|---|
| | PCT/ES2017/070181 |

**A. CLASSIFICATION OF SUBJECT MATTER**

*A61B5/02* (2006.01)
*G06F19/00* (2011.01)
According to International Patent Classification (IPC) or to both national classification and IPC

**B. FIELDS SEARCHED**

Minimum documentation searched (classification system followed by classification symbols)
A61B, G06F

Documentation searched other than minimum documentation to the extent that such documents are included in the fields searched

Electronic data base consulted during the international search (name of data base and, where practicable, search terms used)

EPODOC, INVENES, WPI, NPL, INSPEC, BIOSIS, MEDLINE

**C. DOCUMENTS CONSIDERED TO BE RELEVANT**

| Category* | Citation of document, with indication, where appropriate, of the relevant passages | Relevant to claim No. |
|---|---|---|
| X | WO 2010145009 A1 (HEART FORCE MEDICAL) 23/12/2010, paragraph [1]; paragraphs [9 - 11]; paragraph [43]; paragraph [79]; paragraph [90]; paragraph [110]; paragraph [117]; | 1-10 |
| X | WO 2009073982 A1 (HEART FORCE MEDICAL) 18/06/2009, paragraph [1]; paragraph [15]; paragraphs [81 - 82]; paragraph [96]; paragraph [100]; paragraph [105]; figure 13, | 1-10 |
| A | CN 104424488 A (SHANGHAI ENGINEERING RESEARCH CENTER FOR BROADBAND TECHNOLOGIES & APPLICATIONS) 18/03/2015, paragraphs [3 - 15]; paragraphs [28 -52]; | 1-10 |
| A | CN 102688023 A (TSINGHUA UNIVERSITY) 26/09/2012, paragraphs [9 - 18]; paragraphs [28 - 62]; | 1-10 |

☒ Further documents are listed in the continuation of Box C.    ☒ See patent family annex.

| * | Special categories of cited documents: |
|---|---|
| "A" | document defining the general state of the art which is not considered to be of particular relevance. |
| "E" | earlier document but published on or after the international filing date |
| "L" | document which may throw doubts on priority claim(s) or which is cited to establish the publication date of another citation or other special reason (as specified) |
| "O" | document referring to an oral disclosure use, exhibition, or other means. |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other documents , such combination being obvious to a person skilled in the art |
| "&" | document member of the same patent family |

| Date of the actual completion of the international search | Date of mailing of the international search report |
|---|---|
| 20/06/2017 | **(23/06/2017)** |

| Name and mailing address of the ISA/ | Authorized officer |
|---|---|
| OFICINA ESPAÑOLA DE PATENTES Y MARCAS<br>Paseo de la Castellana, 75 - 28071 Madrid (España)<br>Facsimile No.: 91 349 53 04 | A. Cárdenas Villar<br><br><br>Telephone No. 91 3495393 |

Form PCT/ISA/210 (second sheet) (January 2015)

## INTERNATIONAL SEARCH REPORT

| International application No. |
| --- |
| PCT/ES2017/070181 |

**C (continuation).** DOCUMENTS CONSIDERED TO BE RELEVANT

| Category * | Citation of documents, with indication, where appropriate, of the relevant passages | Relevant to claim No. |
| --- | --- | --- |
| A | ES 2398542 A2 (U. CATALUNYA) 20/03/2013, page 2, lines 5 - 8; page 7, line 14 – page 10, line 28; | 1-10 |
| A | WO 2013109188 A1 (AGENCY FOR SCIENCE, TECHNOLOGY AND RESEARCH) 25/07/2013, paragraphs [20 - 29]; | 1-10 |
| A | US 2010094147 A1 (INAN ET AL.) 15/04/2010, paragraph [2]; paragraphs[7 - 10]; paragraph [56];  paragraph [61]; | 1-10 |
| A | WO 2012103296 A2 (U. LELAND STANFORD JUNIOR) 02/08/2012, the whole document | 1-10 |

Form PCT/ISA/210 (continuation of second sheet) (January 2015)

## INTERNATIONAL SEARCH REPORT

Information on patent family members

International application No.

PCT/ES2017/070181

| Patent document cited in the search report | Publication date | Patent family member(s) | Publication date |
|---|---|---|---|
| WO2010145009 A1 | 23.12.2010 | US2013109989 A1 | 02.05.2013 |
| | | CN102458237 A | 16.05.2012 |
| | | CA2765358 A1 | 23.12.2010 |
| | | EP2442710 A1 | 25.04.2012 |
| WO2009073982 A1 | 18.06.2009 | BRPI0819384 A2 | 05.05.2015 |
| | | RU2010128724 A | 20.01.2012 |
| | | US2011263994 A1 | 27.10.2011 |
| | | CN101951831 A | 19.01.2011 |
| | | CN101951831B B | 22.01.2014 |
| | | CA2709172 A1 | 11.06.2009 |
| | | WO2009073987 A1 | 18.06.2009 |
| | | WO2009073986 A1 | 18.06.2009 |
| | | EP2231000 A1 | 29.09.2010 |
| CN104424488 A | 18.03.2015 | NONE | |
| CN102688023 A | 26.09.2012 | CN102688023B B | 16.10.2013 |
| ES2398542 A2 | 20.03.2013 | WO2013017717 A2 | 07.02.2013 |
| | | WO2013017717 A3 | 28.03.2013 |
| WO2013109188 A1 | 25.07.2013 | SG11201404113T A | 30.10.2014 |
| | | US2015018637 A1 | 15.01.2015 |
| | | CN104114084 A | 22.10.2014 |
| US2010094147 A1 | 15.04.2010 | US2016095521 A1 | 07.04.2016 |
| | | US2015257680 A1 | 17.09.2015 |
| | | US9215991 B2 | 22.12.2015 |
| | | US2015073234 A1 | 12.03.2015 |
| | | US9055871 B2 | 16.06.2015 |
| | | US2014142437 A1 | 22.05.2014 |
| | | US8858449 B2 | 14.10.2014 |
| | | CA2740420 A1 | 22.04.2010 |
| | | AU2009305675 A1 | 22.04.2010 |
| | | WO2010045455 A1 | 22.04.2010 |
| | | EP2348996 A1 | 03.08.2011 |
| | | EP2348996 A4 | 04.06.2014 |
| | | US8870780 B2 | 28.10.2014 |
| WO2012103296 A2 | 02.08.2012 | JP2016101504 A | 02.06.2016 |
| | | JP6130474B B2 | 17.05.2017 |
| | | US2016095522 A1 | 07.04.2016 |
| | | US2015282718 A1 | 08.10.2015 |
| | | US9241637 B2 | 26.01.2016 |
| | | JP2014507213 A | 27.03.2014 |
| | | JP5955341B B2 | 20.07.2016 |
| | | US2013310700 A1 | 21.11.2013 |
| | | US9011346 B2 | 21.04.2015 |
| | | CA2825405 A1 | 02.08.2012 |
| | | AU2012211300 A1 | 09.05.2013 |

Form PCT/ISA/210 (patent family annex) (January 2015)

## INTERNATIONAL SEARCH REPORT

Information on patent family members

International application No.

PCT/ES2017/070181

| Patent document cited in the search report | Publication date | Patent family member(s) | Publication date |
|---|---|---|---|
| | | EP2667769 A2 | 04.12.2013 |
| | | EP2667769 A4 | 22.10.2014 |
| ----------------------------------------------------- | ----------------------- | ----------------------- | -------------- |

Form PCT/ISA/210 (patent family annex) (January 2015)

**EP 3 492 004 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **S. S. AHMED ; G. E. LEVINSON ; C. J. SCHWARTZ ; P. O. ETTINGER.** Systolic Time Intervals as Measures of the Contractile State of the Left Ventricular Myocardium in Man. *Circulation*
- **C. VLACHOPOULOS ; K. AZNAOURIDIS ; C. STEFANADIS.** Prediction of Cardiovascular Events and All-cause Mortality With Arterial Stiffness: a Systematic Review and Meta-analysis. *Journal American College Cardiology*
- **D. BUXI ; J. M. REDOUTÉ ; M. R. YUCE.** A Survey on Signals and Systems in Ambulatory Blood Pressure Monitoring Using Pulse Transit Time. *Physiological Measurements*
- **A. M. WEISSLER ; W. S. HARRIS ; C. D. SCHOEN-FELD.** Systolic Time Intervals in Heart Failure in Man. *Circulation*
- **L. JENSEN ; J. YAKIMETS ; K. K. TEO.** A Review of Impedance Cardiography. *Hear. Lung J. Acute Crit. Care*
- **O. T. INAN ; P. F. MIGEOTTE ; K.-S. PARK ; M. ETEMADI ; K. TAVAKOLIAN et al.** Ballistocardiography and Seismocardiography: a Review of Recent Advances. *IEEE Journal of Biomedical Health and Informatics*